# EUROPEAN PATENT SPECIFICATION

(11) **EP 2 580 597 B1**
(45) Date of publication and mention of the grant of the patent: **07.01.2015**
(21) Application number: 11735547.9
(22) Date of filing: 10.06.2011
(51) Int. Cl.: G01N 33/68

(54) **METHODS AND KITS FOR THE EVALUATION OF A RUMINANT'S DIET**
VERFAHREN UND KITS ZUR BEURTEILUNG DER ERNÄHRUNG VON WIEDERKÄUERN
MÉTHODES ET KITS D'ÉVALUATION DE L'ALIMENTATION D'UN RUMINANT

(30) Priority: 10.06.2010 IT RM20100317
(43) Date of publication of application: 17.04.2013
(73) Proprietor: Universita' Cattolica Del Sacro Cuore, 20123 Milano Mi (IT); Istituto Zooprofilattico Sperimentale Della Lombardia e Dell'Emillia Romagna "Bruno Ubertini" - Izsler, 25124 Brescia (BS) (IT)
(72) Inventor: BERTONI, Giuseppe, I-29122 Piacenza PC (IT); TREVISI, Erminio, I-29122 Piacenza PC (IT); AMADORI, Massimo, Via Antonio Bianchi 9 I-25124 Brescia BS (IT)
(74) Representative: Predazzi, Valentina
(86) International application number: PCT/IB2011/052540
(87) International publication number: WO 2011/154924

(56) References cited:
- SZYMANSKA-CZERWINSKA M ET AL: "Effect of tylosin and prebiotics on the level of cytokines and lymphocyte immunophenotyping parameters in calves", CENTRAL-EUROPEAN JOURNAL OF IMMUNOLOGY 2009 TERMEDIA PUBLISHING HOUSE LTD. POL, vol. 34, no. 1, 2009, pages 1-6, XP002620545, ISSN: 1426-3912
- DSHUROV A: "[Pathomorphologic changes in the ruminal acidosis of sheep].", ARCHIV FÜR EXPERIMENTELLE VETERINÄRMEDIZIN 1976 LNKD- PUBMED:1020999, vol. 30, no. 6, 1976, pages 881-887, XP008132744, ISSN: 0003-9055

## Description

### DESCRIPTION

The present description relates to an *in vitro* method and a kit for the evaluation of a ruminant's diet by a step in which leukocyte and/or cytokine concentration is determined in a rumen fluid sample.

### STATE OF THE PRIOR ART

Ruminants should have a suitable and balanced diet in order to avoid health problems, growth delays or various disorders. In ruminants, overall energy metabolism, as well as use of individual components (proteins, hydrocarbons, lipids) of the diet, goes through a complex cycle of fermentation-degradation of such substrates and the production of important intermediate metabolites, such as volatile short chain fatty acids. A shift in the balance among various components of the food ration entails significant alterations of biofermentative processes in the rumenal environment. Such alterations can also entail consequences at the clinical level, especially under conditions of extreme criticality of nutritional requirements of the animals, as in the case of a dairy cow in the transition period. In fact, in this field an unstable balance subsists, outside of which the two opposite phenomena of acidosis and (subclinical and clinical) alkalosis can intervene with quite serious consequences on the production career of the animals. Besides such phenomena, hoof-located inflammatory alterations, even serious ones, can appear shortly after onset of the above-mentioned rumenal imbalances (Nocek, 1997, J. Dairy Sci. 80:1005-1028).

Alterations of the dietary balance have been associated to the determining of rumenal parameters like pH and concentration of free LPS (lipopolysaccharides), which presumably tend to increase following enhanced autolysis of bacterial cells in rumen fluids (Gozho G.N. et al., 2007, J. Dairy Sci. 90:856-866), whereas there are no studies demonstrating how by determining these parameters the dietary profile could be evaluated.

In the publication of Trevisi et al. J Anim Sci 2009, the Authors describe the results of a study aimed at clarifying the metabolic changes induced by a treatment of some dairy cows with low doses of orally administered interferon-alpha, and report the presence of leukocytes in the rumen fluid.

Szimanzka-Czerwinska et al, Central-European journal of immunology 2009, pages 1-6 describe a method for evaluating a ruminant diet by measuring the level of leucocytes and cytokines in a serum sample taken from the ruminant. Dshurov A, Archiv fur experimentelle veterinärmedizin 1976, pages 881-887 describes the measurement of leucocytes in tissue section taken from the rumen of deceases sheep suffering from acidosis.

The lack of strategies for predicting and monitoring alterations of the dietary balance, which in ruminants can cause serious pathologies, leads to the need to single out methods allowing the evaluation of dietary balances in order to define the diet most suitable to maintain or re-establish the dietary balance.

### SUMMARY OF THE INVENTION

The present description relates to an *in vitro* method and a kit for the evaluation of a ruminant's diet. As it transpires from the prior art, to date it is not possible to evaluate whether a given diet, or dietary profile, could cause an alteration of the dietary balance. The Authors have discovered that the immune system of ruminants is able to react to a "dangerous" (unsuitable, unhealthy, allergenic, unbalanced) qualitative-quantitative composition of the food ration by an increase of rumen fluid colonization by leukocytes. An increase of lymphocyte colonization can also be matched by an increase of cytokine concentration in the rumen fluid. Determining the increase of the concentration of leukocytes and/or cytokines in the rumen fluid can therefore be used in an *in vitro* method for evaluating the correct contribution of food rations administered to ruminants.

Object of the present invention is an *in vitro* method for the evaluation of a ruminant's diet, comprising one or more steps in which leukocyte and/or cytokine concentration is determined in a rumen fluid sample of said ruminant, whereby an increase of the concentration of said leukocytes and/or cytokines in the analyzed sample compared to a baseline value or a value determined in an analogous previous step indicates an unsuitability of the diet. Object of the present invention is also a kit for the evaluation of a ruminant's diet, comprising one or more aliquots of one or more reagents needed for determining leukocyte and/or cytokine concentration in a rumen fluid sample and one or more aliquots of a negative control and/or one or more aliquots of a positive control, wherein said controls comprise a rumen fluid sample with a known concentration of leukocytes and/or T lymphocytes and/or granulocytes and/or cytokines.

Still further advantages, as well as the features and the operation steps of the present invention will be made apparent in the following detailed description of some embodiments thereof, given by way of example and not for limitative purposes.

### DETAILED DESCRIPTION OF THE FIGURE

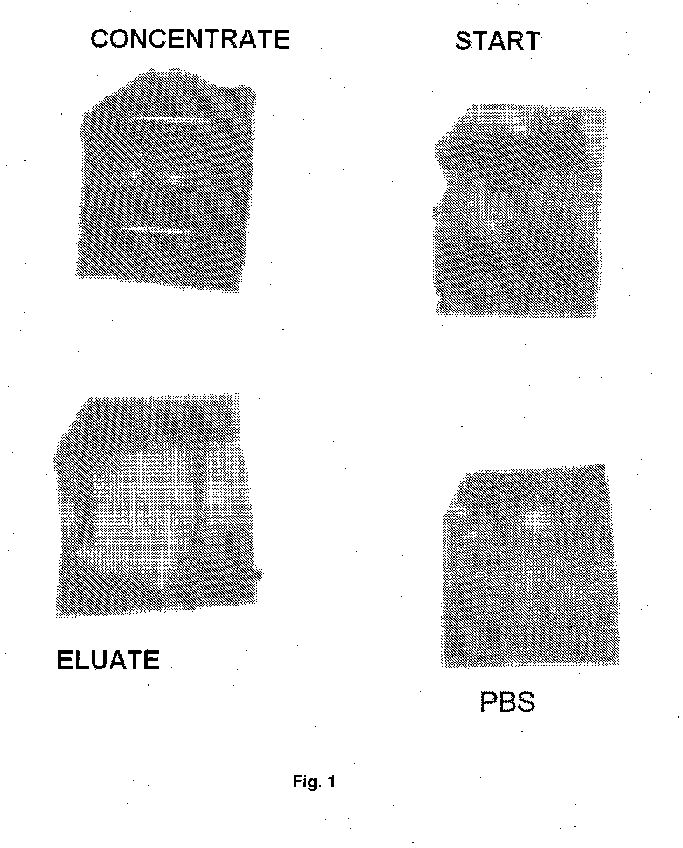

**Figure 1****.** A rumen fluid sample from fistulated heifer was subjected to centrifugation (10.000 rpm, 20 minutes, 4°C). The supernatant was added with PMSF as antiprotease factor and concentrated 25X through an ultrafiltration membrane having a molecular weight cut-off of 10.000 Dalton. Pre- and posttreatment samples were subjected to slot blot on nitrocellulose and bovine IFN-gamma was detected with monoclonal antibody (biotinylated Monoclonal Antibody Clone CC302, code MCA1783b, AbD Serotec Company, Kidlington, UK) + streptavidin-peroxidase conjugate. The reaction was developed with ECL chemiluminescence method (GE Healthcare). Start: rumen fluid before concentration. Concentrate: fluid concentrated 25X by ultrafiltration. Eluate: proteins passed through the membrane. PBS: nitrocellulose adsorbed with buffer alone (negative control).

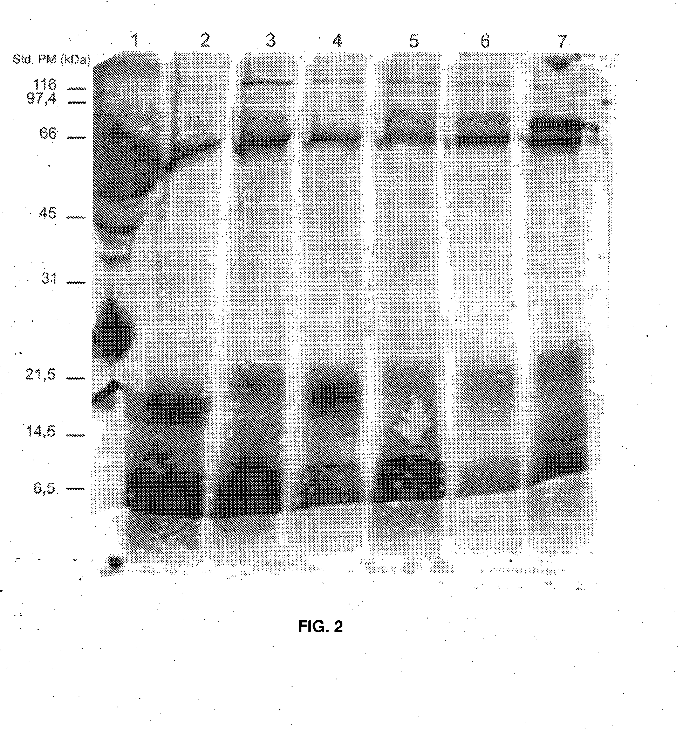

**Figure 2****.** 6 rumen fluid samples from fistulated heifer were subjected to centrifugation (10.000 rpm, 20 min, 4°C) and subsequent concentration by precipitation with 8 volumes of (-20°C) acetone. The same treatment was applied to IFN-gamma-containing bovine plasma. On the next day, precipitated samples were subjected to SDS-PAGE on 4-12.5% gel under reducing conditions, along with protein standards of known molecular weight. Proteins were then transferred on nitrocellulose and bovine IFN-gamma was detected with monoclonal antibody (biotinylated Monoclonal Antibody Clone CC302, code MCA1783b, AbD Serotec Company, Kidlington, UK) + streptavidin-peroxidase conjugate. The reaction was developed with ECL chemiluminescence method (GE Healthcare).
   Sample 1: IFN-gamma-containing bovine plasma. Samples 2-7: concentrated rumen fluids.

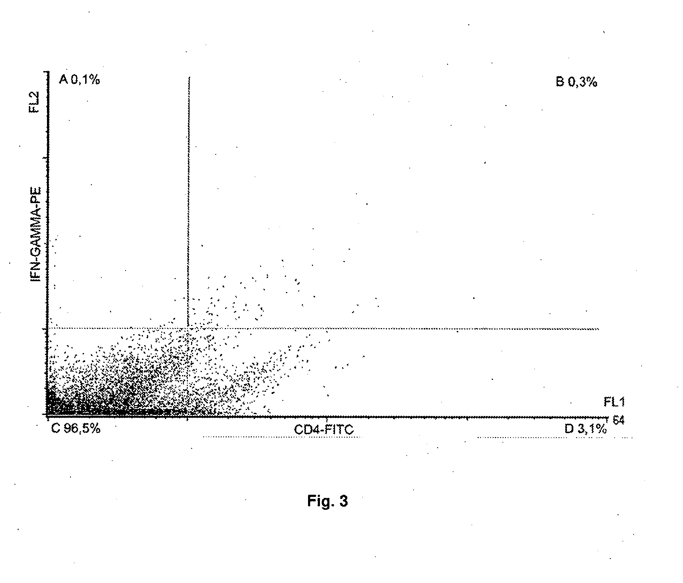

**Figure 3****.** A rumen fluid sample (bovine 3) was treated with PMA+ ionomicyn + brefeldin A (IFN-gamma induction), passed on a Ficoll gradient (1.083), frozen in DMSO and then subjected to rapid thawing at 38°C. After fixation in 4% paraformaldehyde and permeabilization in 0.1% saponine, it was labelled first with anti-bovine IFN-gamma-phycoerythrin (PE) monoclonal antibody and then with anti-CD4 (bovine T lymphocytes) monoclonal antibody. Results refer to cells set in the usual reading channel for bovine leukocytes.

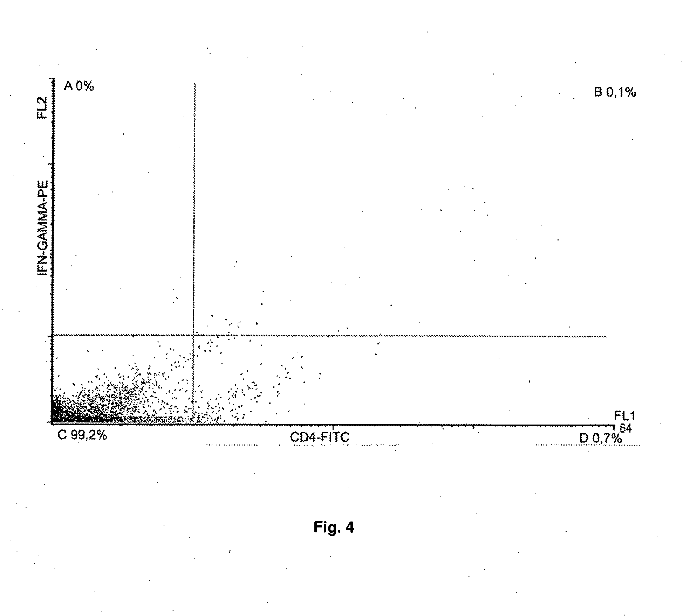

**Figure 4****.** A rumen fluid sample (bovine 7) was treated with PMA+ ionomicyn + brefeldin A (IFN-gamma induction), passed on a Ficoll gradient (1.083), frozen in DMSO and then subjected to rapid thawing at 38°C. After fixation in 4% paraformaldehyde and permeabilization in 0.1% saponine, it was labelled first with anti-bovine IFN-gamma-phycoerythrin (PE) monoclonal antibody and then with anti-CD4 (bovine T lymphocytes) monoclonal antibody. Results refer to cells set in the usual reading channel for bovine leukocytes.

### DETAILED DESCRIPTION OF THE INVENTION

The present description provides an *in vitro* method for the evaluation of a ruminant's diet comprising one or more steps in which leukocyte and/or cytokine concentration is determined in a rumen fluid sample of said ruminant.

Object of the method described herein is to evaluate whether the dietary profile (diet) selected for a given ruminant is suitable (adequate) and therefore does not cause the development of an alteration of the dietary balance. In particular, the method claimed herein constitutes an important tool available to the technician in the field, as it allows to single out the most suitable dietary profile. Moreover, said method also allows to monitor, i.e. to control over time, the response to a given diet of the animal.

By the wording "dietary profile", in the present description it is understood the set of foods constituting the ruminant's diet or food ration. Examples of specific dietary profiles comprise highly fermentiscible diets in which starches constitute about 24% of the dry matter, hyperprotein diets in which proteins constitute about 17% of the dry matter and control diets in which proteins constitute about 9%, and starches and sugars about 10% of the dry matter. An excess of acidogenic (chlorine, sulphur, phosphor) or alkalogenic (sodium, potassium, calcium) elements in the dry matter of the ration will lead respectively to an acidogenic or alkalogenic diet.

The method of the present description may be used with all animal genera and species belonging to the suborder of Ruminants *(Ruminantia),* like e.g. bovines, preferably cows, buffaloes, bisons, ovines, preferably sheep, mouflons, goats, or cervids, giraffids, etc.

By the wording "alterations of the dietary balance" in the present description it is meant the ensemble of dietary disorders, due to qualitative-quantitative imbalance of food ration components with respect to animals' nutritional needs and/or their non-adequately fractionated administration, which can cause disorders of various type and different extent; e.g., the main ones are: digestive; digestive-metabolic; metabolic; immunosuppressing, secretory; reproductory; locomotor.

Rumen fluid samples can be obtained, e.g., by permanent fistula or rumenocentesis. After collection, said samples are kept preferably under refrigeration conditions, in the dark and limiting any aeration phenomena. Samples can be processed on the same day or frozen at the competent laboratory according to appropriate procedures. In this description the technician in the field needs no further information related to various techniques for preserving biological material, since he/she will be able to choose without any effort of inventive activity the one best suited to his/her needs, by considering the detailed information reported in any laboratory manual.

Information related to the evaluation of a diet (dietary profile) are obtained by an analysis of the concentration, in the rumen fluid sample, of leukocytes, preferably T lymphocytes or granulocytes, and/or of cytokines, preferably IFN-gamma, as a privileged index of imbalances in the proflogistic direction triggered by unsuitable diets.

The evaluation of the dietary profile will be based on the increase of leukocyte and/or cytokine concentration in the analyzed sample at a given step compared to the baseline value; baseline value is defined as leukocyte and/or cytokine concentration in a rumen fluid sample of said ruminant determined in a step n-x, where x is greater or equal to 1.

E.g., said baseline value could be the leukocyte and/or cytokine concentration in the ruminant's rumen fluid before a variation in its dietary profile, or the value obtained after a given period of time in which a given dietary profile has been administered to the ruminant, e.g., after 1,2,4,6,7,8,10,20, n days.

In particular, a negative judgment on the dietary profile adopted will be formulated in the presence of a significant increase, preferably at least of a factor 3, of the concentration of leukocytes, preferably of T lymphocytes and/or granulocytes and/or of cytokines (preferably IFN-gamma) compared to the baseline value.

If the analyzed sample has been collected on the occasion of clinical checks in the field relative to anamnestic data of dietary profile alterations, concentration data could be interpreted in a form obviously unlinked to the baseline value, i.e. in the absence of a sample collected before the onset of symptoms of the dietary disorders. In that case, a negative judgment on the dietary profile will be formulated in the presence of at least one of the following elements: i) presence of multiple leukocyte infiltrates ii) presence of granulo-monocitary cells, iii) presence of a single leukocyte population in a percent greater than 5% with respect to total cells iv) presence of IFN-gamma. Some replicates of the field-collected sample could be frozen and compared, in a subsequent step, to samples frozen after the performing of dietary and/or therapeutic interventions of various type, so as to own samples indicative of the baseline value.

In case of cytofluorimetric techniques, the percent of a single leukocyte population will be calculated as the percent of cells positive for the selected leukocyte marker over the total of cells analyzed; therefore, the value will represent the percent of positive reading events with respect to the total of acquired events (preferably at least 10.000).

On the basis of the foregoing, evidently the evaluation of a ruminant's diet could be made by determining, in one or more rumen fluid samples: the sole leukocyte concentration value, or the sole cytokine concentration value or the value of both, comparing them with a baseline value of the same as defined above. The determining could be assisted by the usual physico-chemical analyses of the rumen fluid, like e.g. the pH.

In general, to the ends of the present description it is to be considered suitable any method known to the technician in the field allowing to determine the concentration of the leukocyte population and/or of a cytokine in a biological fluid.

In an embodiment of the method described herein, leukocyte concentration in the rumen fluid samples is calculated by isolating the cell component by conventional techniques, like, e.g., gradient centrifugation, and determining the concentration under cytofluorimetry as described hereinafter.

Isolated cells are incubated at suitable temperatures and for suitable times, with any one primary antibody easily available to the technician in the specific field for surface antigens of leukocytes of the ruminant species analyzed.

The details related to primary antibody incubation protocols are widely known to the technician in the field and, in case commercial antibodies are used, reported in the supplier's instructions. Such incubation protocols provide for the use of suitable buffers, like, e.g., PBS, or, in case commercial antibodies are used, of buffers specifically suggested by the producer, as well as for the suitable dilution to be performed. The incubation time of the primary antibody with the cells to be analyzed should be sufficient, as known to the technician in the field, to guarantee bonding between given antigen and antibody and for this purpose could be, e.g., a minimum incubation time of 20 minutes or a maximum incubation time of 1 hour. In case commercial antibodies are used, incubation time is once again indicated by the supplier.

For the determining of infiltrating leukocytes the following monoclonal antibodies could suitably be employed: IL-A30 (anti-bovine IgM) for B lymphocytes (Naessens et al., 1988), IL-A24 for granulocytes and monocytes (Ellis et al., 1987), MM1A (anti-bovine CD3) for T lymphocytes (Davis et al., 1993).

Primary antibody detecting is performed by use of a fluorochrome-labelled secondary antibody selectively recognizing said primary antibody. Preferably, it is the type of animal used for immunization with the epitope of interest (primary antibody) which defines the nature of the secondary antibody. Therefore, e.g., if the immunized animal is the rabbit, the secondary antibody will be an anti-rabbit; if the immunized animal is the sheep, the secondary will be an anti-sheep, if the immunized animal is the goat, the secondary will be an anti-goat, if the immunized animal is the horse, the secondary will be an anti-horse, etc. In a particular embodiment in which the primary antibody is developed in mouse, the secondary antibody, as is well-known to the technician in the field, will be an anti-mouse secondary antibody.

The secondary antibody could be labelled with any fluorochrome commonly used in the labelling of secondary antibodies; in particular, there could be used a fluorochrome selected from the group comprising: hydroxycoumarin, aminocoumarin, methoxycoumarin, Cascade Blue®, Pacific Blue™, Pacific Orange™, Lucifer Yellow, NDB, phycoerythrin (PE), PE conjugates, Texas Red@, Peridinin chlorophyll (PerCP), TruRed, FluorX, fluorescein, BODIPY-FL, TRICT, X-rhodamine, allophycocyanin (APC), APC conjugates, Alexa Fluor®, FITC, Cy3, Cy5, Cy2, Cy7.

The technical details related to incubation of the labelled secondary antibody are well-known to the technician in the field and amply reported in laboratory manuals, or even, in case of commercial antibodies, provided by the producer. Dilution buffers, and also incubation ones, can be simple buffers like, e.g., PBS or any other known buffer suitable for said steps. For commercial antibodies all experimental conditions, such as buffers, dilution, incubation time and temperature, are reported in the supplier's instructions.

The cells, after having been incubated with the primary and the secondary antibodies, are analyzed in a cytofluorimeter to evaluate their fluorescence, inside a suitable reading window comprising the leukocytes of the analyzed species. Thus, the concentration of leukocytic cells and/or T lymphocytes and/or granulocytes in the sample, expressed e.g. as number of cells /ml, could be determined.

As indicated above, the method of the invention could be carried out by measuring cytokine concentration in the rumen fluid.

In an embodiment of the method described herein, cytokine concentration in the rumen fluid sample to be analyzed is determined by using an ELISA-type immunoenzymatic test; for instance, commercial ELISA tests specific for one or more cytokines could be used, such as, e.g., the BOVIGAM® kit by Prionics for bovine IFN-gamma. The technical details related to the performing of the test are well-known to the technician in the field and amply reported in laboratory manuals. For commercial tests, all experimental conditions such as buffers, dilution, incubation time and temperature, and calculation of the concentration, are reported in the supplier's instructions. The concentration of cytokines obtained is usually expressed as ng/ml.

In case both the value of leukocyte concentration in the rumen fluid sample and the value of cytokine concentration in the same rumen fluid are to be determined, these determining could be carried out by using any one method described herein or alternative methods known in the prior art, suitable for carrying out the desired determining.

Moreover, the Authors of the present invention have observed that by subjecting rumen fluid samples to an *in vitro* fermentation, performed under conditions simulating rumenal fermentation and in the presence of a dietary substrate, representative of a ruminant's diet (dietary profile), it is possible to evaluate, on the basis of the change of cytokine concentration in the sample subjected to fermentation, the effect of a given substrate on the dietary balance.

In particular, the Authors have studied the change of cytokine concentration of a bovine rumen fluid sample fermented in parallel in the presence of an acidogenic substrate, of an alkalogenic substrate and of an "unifeed" control substrate of common use for lactating cows. In the first two cases, in which the dietary substrate consisted of an unbalanced dietary profile (acidogenic and alkalogenic substrate), cytokine concentration increased during fermentation, whereas it remained constant in the case in which the rumen fluid had been fermented with the control substrate.

Therefore, in an embodiment of the method described therein, an aliquot of rumen fluid sample could be subjected to fermentation in the presence of a given dietary substrate. The rumen fluid could be diluted before fermentation in a suitable medium, like, e.g., Mc Dougall's artificial saliva. Cytokine concentration could be determined at time 0 and at different fermentation times, e.g. after 4,8,24,28 hours. Such determining could be considered as baseline values with respect to subsequent determining. Therefore, in this embodiment said baseline value could be: a) the value determined before subjecting said sample to fermentation, in time instants preceding said evaluation; or b) a value determined on the sample subjected to fermentation in studies preceding the evaluation of the suitability of the dietary profile (diet). Concentrations thus determined could be used to evaluate a given dietary profile; a negative judgment on the dietary substrate used in the fermentation will be formulated in the presence of an increase, preferably of a factor 3, of the concentration of cytokines, preferably IFN-gamma, during fermentation, and a positive judgment will be formulated in the presence of a constant or lower value.

This embodiment can allow to obtain also a preliminary evaluation of the suitability of a given dietary profile.

The test could be carried out for screening dietary profiles for ruminant farming, even on a large scale; samples could be frozen, e.g., in DMSO.

To evaluate the dietary profile, apart from cytokine concentration before, after and during rumen fluid fermentation, advantageously also leukocyte concentration in the sample could be determined as described in the foregoing.

Object of the present invention is also a kit for the *in vitro* evaluation of a ruminant's diet comprising aliquots of one or more reagents needed for detecting leukocyte and/or cytokine presence in a rumen fluid sample and one or more aliquots of a negative control and/or one or more aliquots of a positive control, wherein said controls comprise a rumen fluid sample with a known concentration of leukocytes and/or T lymphocytes and/or granulocytes and/or cytokines.. Hence, for the first time a tangible tool is provided that can be used to identify the dietary profiles causing an alteration of the dietary balance.

The kit includes one or more aliquots of a negative control and/or one or more aliquots of a positive control of rumen fluid, both for cytokines (in particular IFN-gamma) and for leukocytes.

The positive control will enable to assay the correctness of the procedure performed and any validity of means used, since it could contain a rumen fluid sample comprising a known concentration of leukocytic cells. In particular, the positive control most suitable, yet not therefor limitative of the invention, will be a rumen fluid sample with a concentration of leukocytic cells greater than 100.000 cells/ml of rumen fluid.

By "negative control" it is meant, e.g., any rumen fluid sample with a concentration of leukocytic cells next to zero.

The kit preferably comprises one or more aliquots of an antibody specific for leukocytic cells. To the ends of the present description, any antibody capable of specifically binding a specific antigen of leukocytes and/or T lymphocytes and/or granulocytes could be comprised in the kit claimed herein.

The kit could further comprise one or more aliquots of a fluorochrome-labelled secondary antibody specific for the primary antibody. Such secondary antibody, as is known to the technician in the field and as already highlighted, should be able to specifically recognize the constant portion of the monoclonal antibody used, and for this reason the selection of the type of secondary antibody to be used will depend on the animal immunized with the epitope of interest. Therefore, by way of a non-limiting example of the present description, if the immunized animal will be the rabbit, the secondary antibody will be an anti-rabbit; if the immunized animal will be the sheep, the secondary will be an anti-sheep; if the immunized animal will be the goat, the secondary will be an anti-goat; if the immunized animal will be the horse, the secondary will be an anti-horse.

The secondary antibody could be labelled with any fluorochrome commonly used in the labelling of secondary antibodies, and in particular there could be used a fluorochrome selected from the group comprising: hydroxycoumarin, aminocoumarin, methoxycoumarin, Cascade Blue®, Pacific Blue™, Pacific Orange™, Lucifer Yellow, NDB, phycoerythrin (PE), PE conjugates, Texas Red®, Peridinin chlorophyll (PerCP), TruRed, FluorX, fluorescein, BODIPY-FL, TRICT, X-rhodamine, allophycocyanin (APC), APC conjugates, Alexa Fluor®, FITC, Cy3, Cy5, Cy2, Cy7.

Tests and examples aimed at better illustrating what has been reported in the present description are given below; in no way such examples are to be construed as a limitation of the preceding description and of the claims hereinafter.

### Testing

Rumen fluid samples were collected, from three cows fitted with rumenal fistula and from three dairy cows in a mid-advanced lactation stage, by rumenocentesis technique. In both cases, collection was performed about 3 hours after unifeed or forage distribution. Samples were collected with extreme care, preventing any contact with skin, and in particular with the incised epithelial surface in case of rumenocentesis, of the syringe with which the rumen fluid was collected. Samples were immediately placed in sterile flasks and cooled in an ice water bath. Said samples were analyzed at +4 hours from collection, by cytofluorimetric technique, to check the presence of animal cells. All samples highlighted T lymphocyte populations in the enriched rumenal material, ranging from 0.2 to 0.9% of the total animal-type cells (i.e., 20-90 lymphocytes on 10,000 cells analyzed). Cow 2 among rumenocentesis-collected ones denoted the highest number of T lymphocytes. Said subject also showed granulocyte presence, and was the only one in which an affection was detected (chronic metritis).

The same test was repeated on rumen fluid collected from two rumenally fistulated cows and from 4 cows at the end of their career, at slaughter. In this case as well, rumen fluid collection was performed carefully avoiding contact with rumen epithelium, therefore preventing any possible pollution.

The rumen fluid from fistulated cows and slaughtered cows was immediately refrigerated in ice water for cytofluorimetric analysis and analysis of content of IFN-gamma, a cytokine produced by lymphocytes during a flogistic process. In parallel, but only on two samples of fistulated cows, an *in vitro* fermentation test was set up by using a system of minifermentators (glass bottles having a nominal capacity of 50 ml) kept at 39°C under anaerobiosis. For each rumen sample, a fermentation lasting 16 hours (in duplicate) and a 40-hour fermentation (single) were set up in said minifermentators. In each minifermentator were placed: 1 ml of rumen fluid (stored in anaerobic environment until the moment of its use), 4 ml of McDougall buffer (9.8 g/l of sodium bicarbonate; 9.3 g/l of sodium phosphate bibasic dodecahydrate; 0.57 g/l of potassium chloride; 0.47 g/l of sodium chloride; 0.12 g/l of magnesium sulphate heptahydrate; 0.06 g/l of anhydrous magnesium chloride; 0.04 g/l of calcium chloride), then the environment was saturated with carbon dioxide. The samples (cows 1 and 2) were additioned of: A) Culture medium (negative control). B) Phorbol-myristate-acetate (PMA) + lonomicyn. C) PMA + lonomicyn + Brefeldin A. D) Phytohemagglutinin (PHA)-P. Aliquots B/C/D were provided for IFN-gamma induction, in particular, aliquot C had to be suitable for cytofluorimeter observation. The incubation period, differentiated for PMA and PHA-P at 16 and 40 hours, was set on the basis of previous experiences on bovine peripheral blood leukocytes, which allowed to establish that PMA has a maximum effect in the short period, whereas PHA-P has a late effect in terms of IFN-gamma secretion. After 16 hours at 39°C, aliquot C was subjected to leukocytic cell enrichment on Ficoll gradient 1.083 g/ml and freezing in 10% DMSO. Aliquots A/B were centrifuged and frozen. The same treatment was performed at +40 hours on aliquot D. In the two rumen samples collected from cows fitted with rumenal fistula, the presence of a modest leukocytic population, analogous to that observed in the previous testing, was detected before fermentation. Apart from T lymphocytes, the presence of myeloid series cells was also signalled.

Of the 4 cows collected at slaughter, 3 showed the presence of T lymphocytes; two of them at levels higher than those found in the previous experiences.

From the fermentation test the presence of an interferon-like substance, analogous to bovine IFN-gamma (a known pro-inflammatory cytokine) was detected in both cows. However, in one of these cows the presence was detected only at +16 hours (PMA fraction) from fermentation start; whereas in the other one it was found at +40 hours (PHA-P fraction) from fermentation start. This means that IFN-gamma production is independent of stimulation presence, but is presumably related to the pattern and extent of rumenal fermentations. It is interesting to note that the increase of IFN-gamma in the fermentation solution is strictly correlated to the appearance of T lymphocytes, always in a small number (a few hundreds of cells per ml), ascertained with cytofluorimetric analysis.

### Tests for checking how alterations of ruminants' dietary balance influence

### rumenal fermentations.

a) Investigation on the production of an interferon-like substance by T lymphocytes in the rumen fluid, in connection to the different composition of the diet.
   The test was conducted with minifermentators made of glass bottles having a capacity of about 100 ml. In each unit were inserted as inoculum: an amount of 20 ml of rumen fluid collected from a heifer fitted with rumenal fistula; 80 ml of Mc Dougall's artificial saliva and a different dietary substrate, representative of ruminants' diets. 4 different substrates were tested:
   - control: "unifeed" of common use for lactating cows (thesis 1);
   - acidogenic thesis: thesis 1, with the addition of an equal (1:1) mixture of barley flour, corn and glucose (thesis 2);
   - alkalogenic thesis: thesis 1, with the addition of soybean extraction flour, urea and MgO (thesis 3);
   - load thesis: thesis 1, plus one-half of additions provided for in theses 2 and 3.

Checks were performed at: 0 (zero), +4, +8, +24 and +48 h from fermentation test start. With the exception of time zero, on which there were no replicates, each thesis and each time was carried out in triplicate. pH of each replicate was measured on a small aliquot. Of the remaining material, 1 ml was added with 8 ml of Mc Dougall buffer and 1 ml of glycerol (both filtered with Whatman filter having 0.22 um porosity), then immediately stored at -80°C until IFN-gamma determining.

The results demonstrated the existence of an IFN secretion modulated by the type of dietary substrate and the fermentation length. In particular, IFN-gamma secretion was observed in all 4 theses, but at different hours from fermentation test start: control at +4, +8 and +24 hours; acidogenic at +24 and +48 hours, alkalogenic at +24 hours only, and load at +4 and +48 hours. Therefore, a certain amount of IFN-gamma would be in the rumenal juice, or, alternatively, there would be the possibility of producing IFN-gamma as a result of specific conditions. Such a stimulation might depend on specific regulatory factors, aimed at modulating the flogistic process in the rumen. Such factors would seem to be strictly connected to diet composition and rumenal fermentation pattern, it also in strict connection to the features of the diet.

### b) In vivo test on cows (heifers) fitted with rumenal fistula and fed more or less fermentiscible and more or less high-protein diets.

Three Italian Frisian cows of about 6 years of age and not in production, fitted with rumenal fistula, were used for a dietary test and consequent collection of rumen liquor aimed at checking the effect on IFN-gamma syntheses. According to a Latin square experimental protocol, the cows received 1-week long dietary treatments. Compared diets were:
a. control diet (raw proteins equal to 9% of dry matter (DM), starch and sugars: 10% of DM and energy concentration of about 0.66 UFL/kg of DM);
b. highly fermentiscible diet (with starches equal to about 24% of DM);
c. hyperprotein diet (with raw proteins equal to about 17% of DM).

In the first two days the diet was gradually adapted to one of the 3 theses; then, after other 2 and 5 days respectively, a rumen fluid sample was collected from the fistula, according to the modes described above. An aliquot of said sample was used for determining pH and another aliquot was stored in duplicate at -80°C for cytofluorimetric and IFN-gamma determining. The rumen fluid of these latter two aliquots was filtered through gauze, then to 2 ml thereof an aliquot of 0.4 ml of dimethyl sulfoxide (DMSO) and 1.6 ml fetal bovine serum were immediately added.

The results highlighted that all three cows showed a more or less marked (T and sometimes also B) leukocyte infiltration with the hyperprotein diet, after both +4 and +7 days from diet variation. Control diet also showed, on the occasion of the first collection (+4 days from diet variation) a slight increase of the lymphocyte population (2 cows out of 3), whereas no relevant infiltrations were had with the highly fermentiscible diet. Therefore, it was confirmed that a diet modification can entail a modification of the leukocyte population in the rumen, to which also a production of IFN-gamma is associated.

### c) Lymphocyte isolation

A magnetic separation test with mAb CD2, CD3, WC1 (T lymphocytes) was carried out on the sample that had highlighted the highest content in T lymphocytes and had been stored in duplicate. Cells, after magnetic separation, were cultured in wells with and without absorption of anti-CD3 mAb to solid phase. Cells with leukocyte morphology were actually isolated, together with several contaminants.

These cells did not proliferate in response to Concanavalin A and bovine anti-bovine CD3.

### d) IFN-gamma production □

At least 4 of the 6 samples of the final fermentation test were positive to ELISA test for bovine IFN-gamma. An ultrafiltration test was set up, from which it emerged that the cytokine showed an unusual molecular mass (probably a truncated form). However, bovine IFN-gamma was clearly recognized by biotinylated monoclonal antibody in the slot-blot test on the sample with higher positivity in ELISA. The data was subsequently confirmed by a Western blotting test on all 6 samples. Control sample (IFN gamma from bovine plasma) revealed 46/40 and 25 kDa samples. Rumen fluid samples (far less reactive in the ELISA assay) showed reaction bands corresponding to 1 or 2 high molecular weight (72/79 kDa) precursors + low molecular weight components, even of <13 kDa. These components are probably accountable for the residual reactivity in ELISA, which is lost precisely after sample concentration through ultrafiltration membrane at 10,000 Da cut-off. Instead, reactivity remained unaltered in the ultrafiltrate.

### EXAMPLES

### EXAMPLE 1

### Leucocyte detection in rumen fluid

Rumen fluid samples can be obtained by permanent fistula or rumenocentesis. After collection, these samples are carried under refrigerated conditions and can be processed on the same day or frozen at -80°C in the presence of 40% fetal bovine serum (FSB) and 10% dimethyl sulfoxide (DMSO). Under such conditions stability is good for at least 6 months. The samples, fresh or just defrozen in a water bath at 38°C, are diluted 1:3 in PBS lacking calcium and magnesium ions, and centrifuged at 1600 g (20 min, 20°C) on Histopaque-1083 (Sigma-Aldrich, St Louis, USA) in 15-ml Falcon tubes and a fluid/gradient ratio equal to 1:2. Cells at the top of the gradient are resuspended in 2% PBS, 0.1% FSB, sodium azide (PBS-FSB-A), washed 1x in the same buffer. Cell pellet is then resuspended in 4 ml of the same buffer / gradient used, stratified on 1 ml FSB and centrifuged at 300 rpm for 10 min at 4°C. The pellet of each aliquot is resuspended in 4 ml of PBS-FSB-A buffer, subdivided into 1-ml aliquots, and finally incubated (30 min, 4°C) with 50 µl of buffer (PBS-FSB-A) alone (control) or with the following monoclonal antibodies in PBS-FSB-A, directed against surface antigens of bovine leukocytes: IL-A30, anti-bovine IgM (B lymphocytes, Naessens et al., 1988); IL-A24 (specific for granulocytes and monocytes, Ellis et al., 1987); MM1A, anti-bovine CD3 (T lymphocytes, Davis et al., 1993). After a washing with PBS-FSB-A, cells are incubated again (30 min, 4°C) with a fluorescein conjugate directed against murine IgG, recognizing the monoclonal antibodies bound to the surface of the very few lymphocytes present. After 2 further washings, cells are resuspended in 300 µl of PBS-FSB-A and analyzed by flow cytofluorimetry. The reading window is in FL1, with a suitable gating based on the scatter features of viable mononucleated cells of bovine peripheral blood; such cells are also added, in preliminary assays, to rumen fluid samples treated as above to validate the detection test under cytofluorimetry. 25,000 control cells, and as many treated with monoclonal antibodies, are subjected to analysis. The percentages of detected cells are compared by Fisher's exact test. In the customary working conditions (1-2% of nonspecific staining in FL1) of the present Inventors, a net difference of 0.3% is statistically significant (P< 0.05). This statistical parameter is of fundamental importance in the light of the fact that, notwithstanding the enrichment caused by the step on Histopaque-1083, it is anyhow recorded the presence of very many rumenal cells/ml (in particular *Infusoria* microorganisms such as Flagellates, Ciliates, Amoebas), compared with a few hundreds of leukocytes/ml, when present

### EXAMPLE 2

### Use of the method

Prepare, as described in Example 1, an aliquot of control rumen fluid collected before any variation of diet, and an aliquot at +3 days (1^{st} collection) and +7 days (2^{nd} collection) from introduction of any variation of the dietary profile. Aliquoting and freezing in 10% DMSO.

Analyze prepared samples under cytofluorimetry for the above-described antigenic determinants or, alternatively, with the sole mAb for CD45 (common leukocyte antigen) as a preliminary screening. Moreover, proceed to determining bovine IFN-gamma with at least 10 replicates of the sample to be compared with as many replicates of the conjugated control and a standard curve to be set up with serial dilutions of recombinant or natural bovine IFN-gamma. The number of replicates indicated is functional to highlight a statistic significance of the difference between the two means, to be determined by a simple *t* test for unpaired data. For this purpose, it is suggested to always eliminate the varying *minus* and *plus* of the series of data obtained, and check the congruence of controls internal to the test (peroxidase-conjugated control, reaction substrate control, positive control, standard curve). Check that: 1) a statistically significant difference exists between the mean of the replicates of the sample and that of the replicates of the conjugated control; 2) the mean optical density (DO) of the replicates falls within the standard cytokine curve.

### EXAMPLE 2

### Use of the method by in vitro fermentators

The analytical procedures of example 2 are suitably carried out also *in vitro*, in a suitable rumen fluid fermentator. In that case, the different experimental theses corresponding to the dietary profiles to be analyzed are analyzed in distinct replicates of the biofermentation units and the control at time 0 will suitably be joined by i) a thesis in which the rumen fluid is simply buffer-diluted, and ii) a thesis in which the rumen fluid is incubated with a certainly non-stressogenic dietary substrate.

### BIBLIOGRAPHY

1 Davis, W. C., N. D. MacHugh, Y. H. Park, M. J. Hamilton, C. R. Wyatt. 1993. Identification of a monoclonal antibody reactive with the bovine orthologue of CD3 (BoCD3). Vet. Immunol. Immunopathol. 39:85-91.
2 Ellis, J. A., W. I. Morrison, B. M. Goddeeris, D. L. Emery. 1987. Bovine mononuclear phagocytic cells: identification by monoclonal antibodies and analysis of functional properties. Vet. Immunol. Immunopathol. 17:125-134.
3 Meager, A., 1987. Quantification of interferons by anti-viral assays and their standardization. Pages 129-147 in Lymphokines and interferons a practical approach. M.J. Clemens, A.G. Morris, A.J.H. Gearing, eds. IRL Press Limited, Oxford, UK.
4 Naessens, J., J. Newson, D. J. Williams, V. Lutje. 1988. Identification of isotypes and allotypes of bovine immunoglobulin M with monoclonal antibodies. Immunology 63:569-574.

## Claims

1. An *in vitro* method for the evaluation of a ruminant's diet, comprising one or more steps in which leukocyte and/or cytokine concentration is determined in a rumen fluid sample of said ruminant, whereby an increase of the concentration of said leukocytes and/or cytokines in the analyzed sample compared to a baseline value or a value determined in an analogous previous step indicates an unsuitability of the diet.

2. The method according to claim 1, wherein said leukocytes are T, B lymphocytes, monocytes and/or granulocytes.

3. The method according to claims 1 or 2, wherein said cytokines are IFN-gamma or other inflammatory cytokines.

4. The method according to any one of the claims 1-3, comprising the following steps:
a) incubating a sample of cells isolated from said rumen fluid sample with a primary leukocyte-specific antibody;
b) incubating with a fluorochrome-labelled secondary antibody specific for the primary antibody the cells obtained after the incubation of step a);
c) evaluating the percent of fluorescent cells of the sample obtained after the incubation of step b) by cytofluorimetry;
d) calculating the concentration of said leukocytes and/or T lymphocytes and/or granulocytes in said rumen fluid sample based on the fluorescence obtained in step c).

5. The method according to the preceding claim, further comprising at least one step wherein the concentration of said cytokines in said rumen fluid sample is determined with an immunoenzymatic test.

6. The method according to any one of the claims 1 to 3, wherein the concentration of said cytokines in said rumen fluid sample is determined with an immunoenzymatic test.

7. The method according to any one of the claims 1 to 6, wherein said baseline value is the concentration of leukocytes or cytokines in a rumen fluid sample of said ruminant before a variation in the diet of said ruminant.

8. The method according to any one of the claims 1 to 6, wherein said analyzed sample is analyzed at step *n* and wherein said baseline value is the concentration of leukocytes or cytokines in a rumen fluid sample of said ruminant determined in a step *n-x,* where x is greater or equal to 1.

9. The method according to any one of the claims 1, 3, 5-8, wherein said rumen fluid sample is subjected to fermentation in the presence of a dietary substrate, wherein said baseline value is the value determined before subjecting said sample to fermentation or a value determined on said sample subjected to fermentation in time instants preceding said evaluation.

10. A kit for the *in vitro* evaluation of a ruminant's diet comprising one or more aliquots of the reagents needed for determining leukocyte and/or cytokine concentration in a rumen fluid sample and one or more aliquots of a negative control and/or one or more aliquots of a positive control, wherein said controls comprise a rumen fluid sample with a known concentration of leukocytes and/or T lymphocytes and/or granulocytes and/or cytokines.

11. The kit according to claim 10, comprising a primary antibody specific for surface antigens of leukocytes and/or T lymphocytes and/or granulocytes.

12. The kit according to any one of the claims 10 to 11, further comprising one or more aliquots of a secondary antibody labelled with a compound selected from the group comprising: hydroxycoumarin, aminocoumarin, methoxycoumarin, Cascade Blue®, Pacific Blue™, Pacific Orange™, Lucifer Yellow, NDB, phycoerythrin (PE), PE conjugates, Texas Red®, Peridinin chlorophyll (PerCP), TruRed, FluorX, fluorescein, BODIPY-FL, TRICT, X-rhodamine, allophycocyanin (APC), APC conjugates, Alexa Fluor®, FITC, Cy3, Cy5, Cy2, Cy7.

13. The kit according to any one of the claims 10-12, wherein said reagents comprise one or more aliquots of buffer solutions and/or one or more aliquots of binding solutions and/or one or more aliquots of reagents for detecting labelled antibodies.

## Patentansprüche

1. In-vitro-Verfahren zur Beurteilung der Ernährung von Wiederkäuern, das umfasst: einen oder mehrere Schritte, in denen die Leukozyten- und/oder Zytokin-Konzentration in einer Pansenflüssigkeitsprobe des Wiederkäuers bestimmt werden, wobei ein Anstieg der Konzentration der Leukozyten und/oder Zytokine in der analysierten Probe im Vergleich zu einem Basiswert oder einem Wert, der in einem analogen früheren Schritt bestimmt wurde, eine Untauglichkeit der Ernährung anzeigt.

2. Verfahren gemäß Anspruch 1, wobei die Leukozyten T-, B-Lymphozyten, Monozyten und/oder Granulozyten sind.

3. Verfahren gemäß den Ansprüchen 1 oder 2, wobei die Zytokine IFN-gamma- oder andere inflammatorische Zytokine sind.

4. Verfahren gemäß einem der Ansprüche 1 - 3, das die folgenden Schritte umfasst:
a) Inkubation einer Probe von Zellen, die aus der Pansenflüssigkeitsprobe isoliert wird, mit einem primären leukozytenspezifischen Antikörper;
b) Inkubation mit einem Fluorochrom-markierten Antikörper, der spezifisch für den ersten Antikörper der Zellen ist, die nach der Inkubation von Schritt a) erhalten werden;
c) Bewertung des prozentualen Anteils von fluoreszierenden Zellen der Probe, der nach der Inkubation des Schrittes b) erhalten wird, durch Zytofluorometrie;
d) Berechnung der Konzentration der Leukozyten und/oder T-Lymphozyten und/oder Granulozyten in der Pansenflüssigkeitsprobe basierend auf der Fluoreszenz, die in dem Schritt c) erhalten wird.

5. Verfahren gemäß dem vorangehenden Anspruch, das weiterhin mindestens einen Schritt umfasst, in dem die Konzentration der Zytokine in der Pansenflüssigkeitsprobe mit einem immunenzymatischen Test bestimmt wird.

6. Verfahren gemäß einem der Ansprüche 1 bis 3, wobei die Konzentration der Zytokine in der Pansenflüssigkeitsprobe mit einem immunenzymatischen Test bestimmt wird.

7. Verfahren gemäß einem der Ansprüche 1 bis 6, wobei der Basiswert die Konzentration von Leukozyten oder Zytokinen in einer Pansenflüssigkeitsprobe des Wiederkäuers vor einer Veränderung der Ernährung des Wiederkäuers ist.

8. Verfahren gemäß einem der Ansprüche 1 bis 6, wobei die analysierte Probe bei dem Schritt n analysiert wird und wobei der Basiswert die Konzentration von Leukozyten oder Zytokinen in der Pansenflüssigkeitsprobe des Wiederkäuers ist, die in dem Schritt n-x bestimmt wird, wobei x größer oder gleich 1 ist.

9. Verfahren gemäß einem der Ansprüche 1, 3, 5 - 8, wobei die Pansenflüssigkeitsprobe einer Fermentation in der Gegenwart eines Nahrungssubstrats ausgesetzt wird, wobei der Basiswert der Wert ist, der bestimmt wird, bevor die Probe einer Fermentation ausgesetzt wird, oder ein Wert, der für die Probe bestimmt wird, die in Zeitpunkten einer Fermentation ausgesetzt wird, die der Beurteilung vorangehen.

10. Kit zur in-vitro-Beurteilung der Ernährung von Wiederkäuern, das umfasst: eine oder mehrere Aliquoten der Reagenzien, die zur Bestimmung einer Leukozyten- und/oder Zytokin-Konzentration in einer Pansenflüssigkeitsprobe benötigt werden, und eine oder mehrere Aliquoten einer negativen Kontrolle und/oder eine oder mehrere Aliquoten einer positiven Kontrolle, wobei die Kontrollen eine Pansenflüssigkeitsprobe mit einer bekannten Konzentration von Leukozyten und/oder T-Lymphozyten und/oder Granulozyten und/oder Zytokinen umfassen.

11. Kit gemäß Anspruch 10, das einen primären Antikörper umfasst, der spezifisch für Oberflächenantigene von Leukozyten und/oder T-Lymphozyten und/oder Granulozyten ist.

12. Kit gemäß einem der Ansprüche 10 bis 11, das weiterhin umfasst: eine oder mehrere Aliquoten eines sekundären Antikörpers, der mit einer der folgenden Verbindungen markiert ist: Hydroxykumarin, Aminokumarin, Methoxykumarin, Cascade Blue®, Pacific Blue™, Pacific Orange™, Lucifer Yellow, NDB, Phycoerythrin (PE), PE-Konjugate, Texas Red®, Peridinin-Chlorophyll (PerCP), Echtrot, FluorX, Fluorescein, BODIPY-FL, TRICT, X-Rhodamin, Allophycocyanin (APC), APC-Konjugate, Alexa Flu- or®, FITC, Cy3, Cy5, Cy2, Cy7.

13. Kit gemäß einem der Ansprüche 10 - 12, wobei die Reagenzien umfassen: eine oder mehrere Aliquoten von Pufferlösungen und/oder eine oder mehrere Aliquote von Bindungslösungen und/oder eine oder mehrere Aliquoten von Reagenzien zur Detektion von markierten Antikörpern.

## Revendications

1. Procédé *in vitro* d'évaluation de l'alimentation d'un ruminant, comportant une ou plusieurs étapes dans laquelle ou lesquelles on détermine une concentration de leucocytes et/ou de cytokines dans un échantillon de liquide ruminal dudit ruminant, étant entendu qu'une augmentation de la concentration desdits leucocytes et/ou cytokines dans l'échantillon analysé, par rapport à une valeur de départ ou à une valeur déterminée dans une étape précédente analogue, indique que l'alimentation ne convient pas.

2. Procédé conforme à la revendication 1, dans lequel lesdits leucocytes sont de lymphocytes T, des lymphocytes B, des monocytes et/ou des granulocytes.

3. Procédé conforme à la revendication 1 ou 2, dans lequel lesdites cytokines sont l'interféron-gamma ou d'autres cytokines inflammatoires.

4. Procédé conforme à l'une des revendications 1 à 3, comportant les étapes suivantes :
a) faire incuber un échantillon de cellules isolées à partir dudit échantillon de liquide ruminal avec un anticorps primaire spécifique des leucocytes ;
b) faire incuber, avec un anticorps secondaire marqué au fluorochrome et spécifique de l'anticorps primaire, les cellules obtenues après l'incubation de l'étape (a) ;
c) évaluer par cytofluorométrie le pourcentage de cellules fluorescentes dans l'échantillon obtenu après l'incubation de l'étape (b) ;
d) et calculer la concentration desdits leucocytes et/ou lymphocytes T et/ou granulocytes dans ledit échantillon de liquide ruminal en se basant sur la fluorescence obtenue dans l'étape (c).

5. Procédé conforme à la revendication précédente, comportant en outre au moins une étape au cours de laquelle on détermine la concentration desdites cytokines dans ledit échantillon de liquide ruminal au moyen d'un test immuno-enzymatique.

6. Procédé conforme à l'une des revendications 1 à 3, dans lequel on détermine la concentration desdites cytokines dans ledit échantillon de liquide ruminal au moyen d'un test immuno-enzymatique.

7. Procédé conforme à l'une des revendications 1 à 6, dans lequel ladite valeur de départ est la concentration de leucocytes ou de cytokines dans un échantillon de liquide ruminal dudit ruminant avant un changement dans l'alimentation dudit ruminant.

8. Procédé conforme à l'une des revendications 1 à 6, dans lequel ledit échantillon est analysé à l'étape n et ladite valeur de départ est la concentration de leucocytes ou de cytokines dans un échantillon de liquide ruminal dudit ruminant, déterminée à l'étape *n-x* où x est supérieur ou égal à 1.

9. Procédé conforme à l'une des revendications 1, 3 et 5 à 8, dans lequel ledit échantillon de liquide ruminal est soumis à une fermentation en présence d'un substrat alimentaire, et dans lequel ladite valeur de départ est la valeur déterminée avant de soumettre ledit échantillon à la fermentation ou une valeur déterminée sur ledit échantillon soumis à la fermentation dans les instants précédant ladite évaluation.

10. Trousse pour l'évaluation *in vitro* de l'alimentation d'un ruminant, comportant une ou plusieurs fractions aliquotes des réactifs nécessaires pour déterminer une concentration de leucocytes et/ou de cytokines dans un échantillon de liquide ruminal et une ou plusieurs fractions aliquotes d'un témoin négatif et/ou une ou plusieurs fractions aliquotes d'un témoin positif, étant entendu que lesdits témoins comprennent un échantillon de liquide ruminal contenant, en une concentration connue, des leucocytes et/ou des lymphocytes T et/ou des granulocytes et/ou des cytokines.

11. Trousse conforme à la revendication 10, comprenant un anticorps primaire spécifique d'antigènes de surface de leucocytes et/ou de lymphocytes T et/ou de granulocytes.

12. Trousse conforme à l'une des revendications 10 et 11, comprenant en outre une ou plusieurs fractions aliquotes d'un anticorps secondaire marqué avec un composé choisi dans l'ensemble des suivants : hydroxy-coumarine, amino-coumarine, méthoxy-coumarine, Cascade Blue^{®}, Pacific Blue^{®}, Pacific Orange^{®}, Lucifer Yellow, NDB, phycoérythrine (PE), conjugués de PE, Texas Red^{®}, Péridinine chlorophylle (PerCP), TruRed, FluorX, fluorescéine, BODIPY-FL, TRICT, X-rhodamine, allophycocyanine (APC), conjugués d'APC, Alexa Fluor^{®}, FITC, Cy3, Cy5, Cy2, Cy7.

13. Trousse conforme à l'une des revendications 10 à 12, dans laquelle lesdits réactifs comprennent une ou plusieurs fractions aliquotes de solutions tampons et/ou une ou plusieurs fractions aliquotes de solutions de liaison et/ou une ou plusieurs fractions aliquotes de réactifs pour détection d'anticorps marqués.
